# EUROPÄISCHE PATENTSCHRIFT

(11) **EP 0 944 643 B1**
(45) Veröffentlichungstag und Bekanntmachung des Hinweises auf die Patenterteilung: **26.02.2003**
(21) Anmeldenummer: 97951267.0
(22) Anmeldetag: 01.12.1997
(51) Int. Cl.: C07J 41/00, A61K 31/58

(54) **OXYIMINOPREGNANCARBOLACTONE**
OXYIMINOPREGNANCARBOLACTONE
OXYIMINOPREGNANCARBOLACTONE

(30) Priorität: 01.12.1996 DE 19651000
(43) Veröffentlichungstag der Anmeldung: 29.09.1999
(73) Patentinhaber: SCHERING AKTIENGESELLSCHAFT, 13353 Berlin (DE)
(72) Erfinder: LAURENT, Henry, D-13467 Berlin (DE); LIPP, Ralph, D-14169 Berlin (DE); ESPERLING, Peter, D-12107 Berlin (DE); TACK, Johannes-Wilhelm, D-13595 Berlin (DE)
(86) Internationale Anmeldenummer: EP9706657
(87) Internationale Veröffentlichungsnummer: WO98024801

(56) Entgegenhaltungen:
- EP-A- 0 709 394
- DE-A- 2 652 761
- DE-A- 3 022 337
- DE-A- 3 916 112
- CHEMICAL ABSTRACTS, vol. 099, no. 7, 15.August 1983 Columbus, Ohio, US; abstract no. 054065, MORAVCSIK I ET AL: "Steroid oxime" XP002061595 & HU 23 286 O (GYOGYSZERKUTATO INTEZET;HUNG.) 30.August 1982

## Beschreibung

Die Erfindung betrifft die neuen Oxyiminopregnancarbolactone der allgemeinen Formel I, ein Verfahren zu deren Herstellung, diese Oxyiminopregnancarbolactone enthaltende pharmazeutische Präparate sowie deren Verwendung zur Herstellung von Arzneimitteln.

Die Erfindung betrifft die *(E,Z)*-Gemische und die isomerenreinen *(E)-* und *(Z)*-Verbindungen der Formel I, 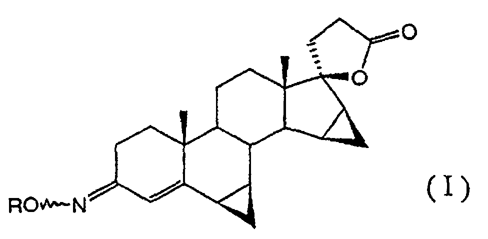 worin
R ein Wasserstoffatom oder einen Acylrest mit 2 bis 10 C-Atomen bedeutet.

Als Acylrest R ist ein Rest C(O)R', worin R' ein gerad- oder verzweigtkettiger oder cyclischer, gesättigter oder bis zu dreifach ungesättigter Kohlenwasserstoffrest, ein Alkylcycloalkyl- oder cycloalkenylrest, jeweils mit bis zu 9 Kohlenstoffatomen, oder ein Benzoylrest ist, geeignet.

Bevorzugte Reste für R sind entweder das Wasserstoffatom oder ein linearer, gesättigter Alkanoylrest mit 2 bis 10 Kohlenstoffatomen, d.h. R' ist eine Methyl-, Ethyl-, Propyl-, Butyl-, Pentyl-, Hexyl-, Heptyl-, Octyl- oder Nonylgruppe.

Als ein verzweigtkettiger, gesättigter Kohlenwasserstoffrest ist beispielsweise der *i*-Propyloder *t*-Butylrest zu nennen.

Als cyclischer Kohlenwasserstoffrest kommen in erster Linie der Cyclopropyl-, Cyclopentyl- oder Cyclohexylrest in Frage.

Der Methylcyclopropyl-, Methylcyclohexyl- oder Methylcyclohexenylrest seien als Vertreter für einen Alkylcycloalkyl- bzw. cycloalkenylrest angeführt.

Die zu den Verbindungen der allgemeinen Formel I analoge 3-Keto-Verbindung der Formel II (Drospirenon) 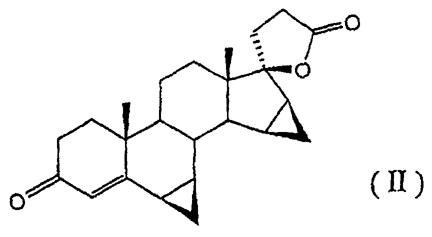 ist als Verbindung mit
a) Antialdosteronwirkung (DE-A 26 52 761)
b) Gestagenwirkung (DE-A 30 22 337) sowie
c) starker antiandrogener Wirkung, und dies bei einer zur Kontrazeption ausreichenden Dosierung (DE-A 39 16 112)
beschrieben.

Drospirenon ist das erste synthetische Gestagen, das, wie das natürliche Progesteron, alle drei Partialwirkungen a), b) und c), und zwar in einem gemeinsamen Dosisbereich, aufweist, im Gegensatz zum Progesteron aber auch nach oraler Gabe in relevanter Menge bioverfügbar wird. Drospirenon kann daher entweder alleine oder bevorzugterweise in Kombinationspräparaten zusammen mit einem Estrogen Verwendung finden zur hormonalen Kontrazeption und/oder zur Hormonersatz-Therapie. Aufgrund der antimineralocorticoiden und antiandrogenen Partialwirkung sind diese Präparate auch für solche Anwenderinnen geeignet, für die hormonale Kombinatonspräparate ansonsten kontraindiziert sind (DE-A 39 16 112).

Die benötigte tägliche Dosismenge Drospirenon zur Kontrazeption oder Hormonersatz-Therapie beträgt 1 bis 10 mg.
Seit einiger Zeit sind auch die transdermale Anwendung sowie die subcutane Applikation durch sogenannte Implants von hormonellen Wirkstoffen zur Hormonersatz-Therapie und neuerdings auch zur Kontrazeption von großem Interesse (Te-Yen Chien et al., "Transdermal Contraceptive Delivery Sytem: Preclinical Development and Clinical Assessment" in Drug Development and Industrial Pharmacy, 20(4), 633 - 664 (1994)).

Einer sinnvollen Verwendung des Drospirenons über die zuletzt genannten Applikationsrouten stehen bisher dessen ungünstige physikochemische Substanzeigenschaften, wie z.B. geringe Löslichkeit in organischen Polymeren, entgegen.

Die Aufgabe der vorliegenden Erfindung besteht daher darin, Drospirenon in Derivate zu überführen, die deutlich verbesserte physikochemische Substanzeigenschaften aufweisen sollen, ohne daß das sehr günstige pharmakologische Profil wesentlich verändert wird.

Es wurde nunmehr gefunden, daß sich dies durch Überführung von Drospirenon in das 3-Oximderivat (R = H) bzw die entsprechenden O-Acylderivate (R = Acyl) der allgemeinen Formel I erreichen läßt. Überraschenderweise zeichnen sich die Derivate der allgemeinen Formel I durch eine mehrfach höhere Löslichkeit als Drospirenon in organischen Polymeren, die als Hauthaftkleber geeignet sind, wie z.B. Polyacrylate, Silikonkleber, Synthesekautschuk) aus. Erst diese stark erhöhte Löslichkeit gestattet bei der transdermalen Anwendung den Austritt der intakten Produgs der allgemeinen Formel I aus der Matrix in einer Menge, durch die sich ein ausreichender transdermaler Fluß der aktiven Verbindung (Drospirenon) oder auch deren Prodrug (Verbindung der allgemeinen Formel I) erzielen läßt. Dies ist wiederum Voraussetzung dafür, daß ein relevanter Wirkstoffspiegel im Serum überhaupt zustande kommt.

Die Verbindungen erlauben es also überhaupt erst, die kontrazeptive oder therapeutische Wirkung von Drospirenon nach transdermaler Applikation eines Prodrugs auszuschöpfen. Ebenso wie Drospirenon selbst können sie aber auch oral gegeben werden.

Kontrazeptiv wirksame 3-Oxime und O-Acylate sind in der 19-Nortestosteron-Reihe bereits beschrieben worden. Levonorgestrel-oxim-17-acetat ist seit einigen Jahren als Kombinationspräparat mit Ethinylestradiol auf dem OC-Markt (DE 16 18 752, DE 16 20 102, DE 26 33 210, US 3 780 073, US 4 027 019, alle Ortho Pharmaceutical Corp.).

Pharmakologisch aktive 3-Oxime und O-Acylate von Steroid-Spirolactonen sind bisher nicht beschrieben worden; lediglich 3-Hydroxyimino-5β,17α-pregnan-21,17-carbolacton geht aus der DE 43 21 937 als Zwischenverbindung zur Herstellung der als zur Therapie der latenten und manifesten Herzinsuffizienz geeigneten entsprechenden 3-Aminoverbindung hervor.

Gegenstand der Erfindung ist auch das Verfahren zur Herstellung der Verbindungen der Formel I.

Die Herstellung der Verbindungen der Formel I ist dadurch gekennzeichnet, daß man die Verbindung der Formel II (Drospirenon) 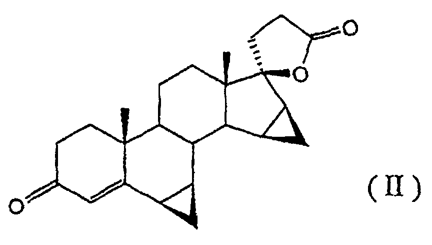 in die 3-Hydroxyiminoverbindungen überführt und gewünschtenfalls diese anschließend durch Veresterung mit einem Carbonsäureanhydrid [(R'C(O)]₂O oder einem Säurehalogenid R'C(O)X (X = Cl, Br; R' hat die in der allgemeinen Formel I angegebene Bedeutung) in Gegenwart einer Base zu den 3-Acyloxyiminoverbindungen umsetzt.

Das Oxim der allgemeinen Formel I (d.h. R = H) entsteht bei der Reaktion von Drospirenon mit Hydroxylamin-Hydrochlorid/Pyridin als (E,Z)-Gemisch mit einem (E,Z)-Verhältnis ≈ 4:1.
Durch Umsetzung mit dem entsprechenden Säureanhydrid oder -halogenid in Gegenwart von Pyridin, gegebenenfalls unter Zusatz von Dimethylaminopyridin, überführt man das (E,Z)-Gemisch des Oxims in die Acyloxyimino-Verbindungen [d.h. R = C(O)R'] der allgemeinen Formel I.

Die in den Verbindungen der Formel I enthaltene C=N-Doppelbindung gibt Anlaß zum Auftreten geometrischer Isomeren in Form von *(E,Z)*-Gemischen, die sich chromatographisch in die reinen (E)- und (Z)-Isomeren auftrennen lassen.

Bei den erfindungsgemäßen Verbindungen handelt es sich um äußerst wirksame Gestagene, die zur Erhaltung von Schwangerschaften bei transdermaler, parenteraler wie auch bei oraler Applikation geeignet sind. In Kombination mit einem Estrogen sind Kombinationspräparate erhältlich, die für die Kontrazeption und bei klimakterischen Beschwerden eingesetzt werden können.

Aufgrund ihrer hohen gestagenen Wirksamkeit können die neuen Verbindungen der allgemeinen Formel (I) beispielsweise allein oder in Kombination mit Estrogenen in Präparaten zur Kontrazeption verwendet werden. Aber auch alle anderen heutzutage für Gestagene bekannten Verwendungsmöglichkeiten stehen den neuen Verbindungen offen (siehe z.B. "Kontrazeption mit Hormonen", Hans-Dieter Taubert und Herbert Kuhl, Georg Thieme Verlag Stuttgart - New York, 1995).

Die Dosierung der Wirkstoffe kann je nach Verabfolgungsweg, Alter und Gewicht des Patienten, der gewünschten Indikation sowie Art und Schwere der zu behandelnden Erkrankung und ähnlichen Faktoren variieren. Die tägliche Dosis beträgt 0.1-25 mg, bevorzugt 0.5-5 mg, wobei die Dosis als einmal zu verabreichende Einzeldosis oder unterteilt in zwei oder mehreren Tagesdosen gegeben werden kann. Im Fall der Transdermalsysteme können auch bis zu 14 Tagesdosen in Folge von einem System abgegeben werden. Im Fall der Implantate, Intravaginalsysteme, wie z.B. einem Vaginalring und Intrauterinsysteme, wie z.B. Mirena, kann die Wirkstoffabgabe über einen Zeitraum von bis zu 3 Jahren erfolgen.

Die gestagenen und estrogenen Wirkstoffkomponenten werden in Kontrazeptionspräparaten vorzugsweise zusammen appliziert. Die tägliche Dosis wird bei oraler Applikation vorzugsweise einmalig verabfolgt.

Als Estrogene kommen alle natürlichen und synthetischen, als estrogen wirksame bekannten Verbindungen in Frage.

Als natürliche Estrogene sind dies insbesondere Estradiol sowie auch dessen länger wirkende Ester wie das Valerat etc. oder Estriol.

Als synthetische Estrogene sind das Ethinylestradiol, 14α,17α-Ethano-1,3,5(10)-estratrien-3,17β-diol (WO 88/01275), 14α,17α-Ethano-1,3,5(10)-estratrien-3,16α, 17β-triol (WO 91/08219) oder die 15,15-Dialkyl-Derivate des Estradiols, und hiervon insbesondere das 15,15-Dimethylestradiol, zu nennen. Als synthetisches Estrogen ist Ethinylestradiol bevorzugt.
Auch die kürzlich bekannt gewordenen Estratrien-3-amidosulfonate (WO 96/05216 und WO 96/05217), abgeleitet vom Estradiol oder Ethinylestradiol, die sich durch geringe hapatische Estrogenität auszeichnen, sind als Estrogene zur gemeinsamen Verwendung mit den Verbindungen der allgemeinen Formel I geeignet.
Schließlich seien noch die 14α,15α-Methylensteroide aus der Estranreihe (US-Patent 4,231,946), insbesondere das 14α,15α-Methylen-17α-estradiol sowie die entsprechenden Ester-Derivate (WO 95/01988) erwähnt.

Das Estrogen wird in einer Menge verabfolgt, die der von 0,01 bis 0,05 mg Ethinylestradiol entspricht.

Die neuen Verbindungen der allgemeinen Formel (I) können auch in Präparaten zur Behandlung gynäkologischer Störungen und zur Substitutionstherapie eingesetzt werden.

Schließlich können die neuen Verbindungen auch als gestagene Komponente in den neuerdings bekannt gewordenen Zusammensetzungen für die weibliche Fertilitätskontrolle, die sich durch die zusätzliche Verwendung eines kompetitiven Progesteronantagonisten auszeichnen, zum Einsatz kommen (H.B. Croxatto und A.M. Salvatierra in Female Contraception and Male Fertility Regulation, ed. by Runnebaum, Rabe & Kiesel - Vol. 2, Advances in Gynecological and Obstetric Research Series, Parthenon Publishing Group - 1991, Seite 245).
Die Dosierung liegt im bereits angegebenen Bereich, die Formulierung kann wie bei konventionellen OC-Präparaten erfolgen. Die Applikation des zusätzlichen, kompetitiven Progesteronantagonisten kann dabei auch sequentiell vorgenommen werden.

Für die transdermale Anwendung sind insbesondere Transdermalsysteme nach dem Matrix- oder Membranprinzip sowie halbfeste und flüssige Zubereitungen wie Oleo- oder Hydrogele geeignet.
Zur Herstellung der Transdermalsysteme eignen sich Hauthaftklebstoffe wie z. B. Polyacrylate, Silikonkleber, synthetische Kautschuke wie Polyisobutylen sowie Folien wie z. B. Polyethylen, Polypropylen, Ethylenvinylacetat, Polyvinylchlorid, Polyvinylidenchlorid, Polyester sowie abziehbare Schutzfolien aus z. B. Polyester oder Papier die ein- oder beidseitig silikonisiert oder fluorpolymerbeschichtet sind.
Zur Herstellung transdermaler Lösungen und Gele eignen sich Wasser und organische Lösungsmittel sowie deren Gemische. Gele können durch das Einarbeiten von entsprechenden Gelbildnern wie z. B. Siliziumdioxid, Traganth, Stärke und deren Derivaten, Cellulose und deren Derivaten oder Polyacrylsäure und deren Derivaten verdickt werden.
Die Formulierung der Verbindungen der allgemeinen Formel I in einem transdermalen System kann analog der in der WO 94/04157 beschriebenen Formulierung von 3-Ketodesogestrel vorgenommen werden.
Zur möglichen Ausgestaltung transdermaler Syteme, in die die Verbindungen der allgemeinen Formel I eingearbeitet werden können, wird beispielsweise auf folgende grundlegenden Literaturstellen verwiesen: Barry, B.W., "Dermatological Formulations, Percutaneous Absorption"; Marcel Dekker, Inc., New York - Basel, 1983 sowie Chien, Y.W., "Transdermal Controlled Systemic Medications", Marcel Dekker, Inc., New York - Basel, 1987.

Für die Herstellung von Implantaten oder arzneistoffbeladenen Intravaginal- (z.B. Vaginalringe) oder Intrauterinsystemen (z.B. Pessare, Spiralen) eignen sich verschiedene Polymere wie zum Beispiel Silikonpolymere, Ethylenvinylacetat, Polyethylen und Polypropylen.

Verbindungen der allgemeinen Formel I enthaltende pharmazeutische Präparate werden nach üblichen Verfahren hergestellt, indem man den Wirkstoff mit geeigneten Träger-, Hilfs- und/oder Zusatzstoffen in die Form eines pharmazeutischen Präparates bringt, das für die enterale oder parenterale Applikation geeignet ist. Die so erhaltenen Zubereitungen können als Arzneimittel in der Human- oder Veterinärmedizin eingesetzt werden. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan, intramuskulär oder intravenös anwendbaren Injektionslösungen oder Implantaten oder topisch oder intrathekal oder transdermal oder mittels Intrauterinpessar erfolgen. Als Hilfsstoffe für die gewünschte Arzneimittelformulierung sind die dem Fachmann bekannten inerten organischen und anorganischen Trägermaterialien geeignet wie z. B. Wasser, Gelatine, Gummmi arabicum, Milchzucker, Stärke, Magnesiumstearat, Talk, Silikonpolymere, Polyacrylate, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form, z. B. als Tabletten, Dragees, Suppositorien, Kapseln, Implantate, Intravaginalsysteme, Intrauterinsysteme, Transdermalsysteme oder in flüssiger Form, z. B. als Lösungen, Suspensionen oder Emulsionen vorliegen.

Als Trägersysteme können auch grenzflächennahe Hilfsstoffe wie Salze, Gallensäuren oder tierische oder pflanzliche Phospholipide und deren Mischungen sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie z. B. Lactose, Mais- oder Kartoffelstärke, geeignet.

Die nachfolgenden Beispiele dienen der näheren Erläuterung der Erfindung:

### Beispiel 1

### 3-Hydroxyimino-6β,7β;15β,16β-dimethylen-17α-pregn-4-en-21,17-carbolacton

A. Eine Lösung von 10.0 g 3-Oxo-6β,7β;15β,16β-dimethylen-17α-pregn-4-en-21,17-carbolacton in 75 ml Pyridin wird mit 3.15 g Hydroxylammoniumchlorid versetz und eine Stunde auf dem Dampfbad erhitzt. Die Reaktionsmischung wird in Eiswasser eingerührt, der ausgefallene Niederschlag wird abfiltriert, mehrmals mit Wasser gewaschen und in Dichlormethan gelöst. Die Lösung wird über Natriumsulfat getrocknet und das Lösemittel im Vakuum abdampft. Der kristalline Rückstand besteht aus 8.91 g *(E,Z)*-3-Hydroxyimino-6β,7β;15β,16β-dimethylen-17α-pregn-4-en-21,17-carbolacton, mit einem *(E,Z)*-Verhältnis von 4:1, Schmelzpunkt 194 °C, [α]_{D} = 174° (CHCl₃).
**B.** 2.0 g *(E,Z)*-3-Hydroxyimino-6β,7β;15β,16β-dimethylen-17α-pregn-4-en-21,17-carbolacton werden an einer Silicagel-Säule (Kromasil 100/10 µm) mit einem Hexan-Ethylacetat-Gemisch (7:3) chromatographiert. Es werden 940 mg *(E)*-3-Hydroxyimino-6β,7β;15β,16β-dimethylen-17α-pregn-4-en-21,17-carbolacton, Schmelzpunkt 244 °C, und 330 mg *(Z)*-3-Hydroxyimino-6β,7β;15β,16β-dimethylen-17α-pregn-4-en-21,17-carbolacton, Schmelzpunkt 156 °C, eluiert.

### Beispiel 2

### 3-Acetoxyimino-6β,7β;15β,16β-dimethylen-17α-pregn-4-en-21,17-carbolacton

**A.** Eine Lösung von 3.0 g *(E,Z)*-3-Hydroxyimino-6β,7β;15β,16β-dimethylen-17α-pregn-4-en-21,17-carbolacton, *(E,Z)*-Verhältnis 4:1, in 15 ml Pyridin wird mit 7.5 ml Acetanhydrid versetzt und 3 Stunden bei Raumtemperatur gerührt. Die Reaktionsmischung wird in Eiswasser eingerührt, der ausgefallene Niederschlag wird abfiltriert, mehrmals mit Wasser gewaschen und in Dichlormethan gelöst. Die Lösung wird über Natriumsulfat getrocknet und das Lösemittel im Vakuum abdampft. Als kristallinen Rückstand erhält man 3.21 g *(E,Z)*-3-Acetoxyimino-6β,7β;15β,16β-dimethylen-17α-pregn-4-en-21,17-carbolacton, Schmelzpunkt 212 °C, [α]_{D} = -187° (CHCl₃).
**B.** 960 mg *(E,Z)*-3-Acetoxyimino-6β,7β;15β,16β-dimethylen-17α-pregn-4-en-21,17-carbolacton werden an einer Silicagel-Säule (Kromasil 100/10 µm) mit einem Hexan-Ethylacetat-Gemisch (7:3) chromatographiert. Es werden 531 mg *(E)*-3-Acetoxyimino-6β,7β;15β,16β-dimethylen-17α-pregn-4-en-21,17-carbolacton, Schmelzpunkt 225 °C, [α]_{D} = -195° (CHCl₃), und 227 mg *(Z)*-3-Acetoxyimino-6β,7β;15β,16β-dimethylen-17α-pregn-4-en-21,17-carbolacton, Schmelzpunkt 230 °C, [α]_{D} = -152° (CHCl₃), eluiert.

### Beispiel 3

### 3-Propionyloxyimino-6β,7β;15β,16β-dimethylen-17α-pregn-4-en-21,17-carbolacton

Eine Lösung von 6.0 g *(E,Z)*-3-Hydroxyimino-6β,7β;15β,16β-dimethylen-17α-pregn-4-en-21,17-carbolacton, *(E,Z)*-Verhältnis = 4:1, in 30 ml Pyridin wird mit 15 ml Propionsäureanhydrid versetzt und 15 Stunden bei Raumtemperatur gehalten. Die Reaktionsmischung wird in Eiswasser gegossen und 2 Stunden gerührt, der ausgefallene Niederschlag wird abfiltriert, mehrmals mit Wasser gewaschen und in Dichlormethan gelöst. Die Lösung wird über Natriumsulfat getrocknet und das Lösemittel im Vakuum abdampft. Als Rückstand erhält man 6.85 g kristallines *(E,Z)*-3-Propionyloxyimino-6β,7β;15β,16β-dimethylen-17α-pregn-4-en-21,17-carbolacton, Schmelzpunkt 172 °C, [α]_{D} = -180° (CHCl₃).

### Beispiel 4

### 3-Butyryloxyimino-6β,7β;15β,16β-dimethylen-17α-pregn-4-en-21,17-carbolacton

Eine Lösung von 6.0 g *(E,Z)*-3-Hydroxyimino-6β,7β;15β,16β-dimethylen-17α-pregn-4-en-21,17-carbolacton, *(E,Z)*-Verhältnis 4:1, in 30 ml Pyridin wird mit 15 ml Buttersäureanhydrid versetzt und 16 Stunden bei Raumtemperatur gehalten. Die Reaktionsmischung wird in Eiswasser gegossen, 3 Stunden gerührt und die sich abscheidende ölige Phase in Dichlormethan gelöst. Die Lösung wird mehrmals mit Wasser gewaschen, über Natriumsulfat getrocknet und das Lösemittel im Vakuum abdampft. Als Rückstand erhält man 5.7 g kristallines *(E,Z)*-3-Butyryloxyimino-6β,7β;15β,16β-dimethylen-17α-pregn-4-en-21,17-carbolacton, Schmelzpunkt 142 °C, [α]_{D} = -179° (CHCl₃).

### Beispiel 5

### 3-Hexanoyloxyimino-6β,7β;15β,16β-dimethylen-17α-pregn-4-en-21,17-carbolacton

Eine Lösung von 4.4 g *(E,Z)*-3-Hydroxyimino-6β,7β;15β,16β-dimethylen-17α-pregn-4-en-21,17-carbolacton, *(E,Z)*-Verhältnis 4:1, in 30 ml Pyridin wird mit 15 ml Capronsäureanhydrid sowie 500 mg 4-Dimethylaminopyridin versetzt und zwei Stunden auf 60 °C erhitzt. Die Reaktionsmischung wird in Eiswasser gegossen, 15 Stunden gerührt und das sich abscheidende ölige Produkt in Dichlormethan gelöst. Die Lösung wird mehrmals mit Wasser gewaschen, über Natriumsulfat getrocknet und das Lösemittel im Vakuum abgedampft. Der Rückstand von 6.13 g wird an einer Silicagel-Säule (Kromasil 100/10µm) mit einem Hexan-Ethylacetat-Gemisch (7:3) chromatographiert. Man eluiert 5.6 g *(E,Z)*-3-Hexanoyloxyimino-6β,7β;15β,16β-dimethylen-17α-pregn-4-en-21,17-carbolacton in Form eines schwach gelben Öls, [α]_{D} = -143° (CHCl₃), sowie 1.32 g *(E)*-3-Hexanoyloxyimino-6β,7β;15β,16β-dimethylen-17α-pregn-4-en-21,17-carbolacton als Schaum, [α]_{D} = -171° (CHCl₃), und 0.33 g *(Z)*-3-Hexanoyloxyimino-6β,7β;15β,16β-dimethylen-pregn-4-en-21,17-carbolacton als Schaum, [α]_{D} = -130° (CHCl₃).

### Beispiel 6

### 3-Nonanoyloxyimino-6β,7β;15β,16β-dimethylen-17α-pregn-4-en-21,17-carbolacton

Eine Lösung von 5.7 g (*E,Z*)-3-Hydroxyimino-6β,7β;15β,16β-dimethylen-17α-pregn-4-en-21,17-carbolacton in 30 ml Pyridin wird mit 5 ml Nonansäureanhydrid versetzt und 30 Minuten auf dem Dampfbad erhitzt. Die Reaktionsmischung wird in Eiswasser eingerührt, nach 30 Minuten wird das ölige Produkt isoliert und in Dichlormethan aufgenommen. Die Lösung wird mehrmals mit Wasser gewaschen, über Natriumsulfat getrocknet und das Lösemittel im Vakuum abgedampft. Der Rückstand wird an einer Silicagel-Säule mit einem Hexan-Ethylacetat-Gemisch (7:3) chromatographiert. Man eluiert 7.8 g *(E,Z)*-3-Nonanoyloxyimino-6β,7β;15β,16β-dimethylen-17α-pregn-4-en-21,17-carbolacton in Form eines schwach gelben Öls, [α]_{D} = -128° (CHCl₃).

### Beispiel 7

0.3 g der Verbindung aus Beispiel 1A. und 2,5 g einer 30%igen Lösung von Kollidon VA 64 in Isopropanol wurden in ein Becherglas eingewogen und 10 min gerührt sowie anschließend 30 min im Ultraschallbad beschallt. Es wurde mit 8,24 g Polyacrylatkleber Gelva 7881 (50%ige Kleberfeststofflösung in Ethylacetat, Solutia, Springfield, MA, USA) dazugewogen und mit Isopropanol zu 15 g aufgefüllt. Es wurde 20 min. ultrabeschallt und 1 h bei 50 °C gerührt. Die erhaltene Mischung wurde mit einer Rakel auf Release Liner (silikonisiertes PET, Bertek, St. Albans, VT, USA) aufgezogen, so daß ein Trockenbeschichtungsgewicht von 80 g/m2 entsteht. Anschließend wurde 20 min. bei 70 °C getrocknet. Polyethylen-Backing-Folie (CoTran 9720, 3M, St. Paul, MN, USA) wurde auflaminiert und mittels einer runden Stanze Einzelpflaster von 20 cm² gestanzt.

**Beispiel 8**

wie Beispiel 7, aber mit 0,3 g der Verbindung aus Beispiel 2B.

## Patentansprüche

1. *(E,Z)*-Gemische und isomerenreine *(E)-* und *(Z)*-Verbindungen der Formel I, 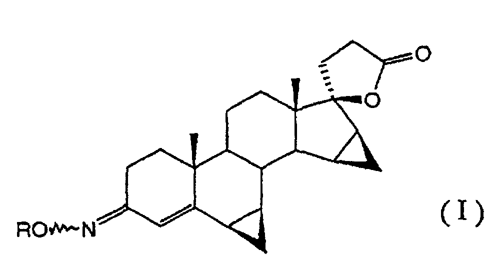 worin
R ein Wasserstoffatom oder einen Acylrest mit 2 bis 10 C-Atomen bedeutet.

2. Verbindungen nach Anspruch 1, **dadurch gekennzeichnet, daß** R ein Wasserstoffatom ist.

3. Verbindungen nach Anspruch 1, **dadurch gekennzeichnet, daß** R ein Rest C(O)R', worin R' ein gerad- oder verzweigtkettiger oder cyclischer, gesättigter oder bis zu dreifach ungesättigter Kohlenwasserstoffrest, ein Alkylcycloalkyl- oder cycloalkenylrest, jeweils mit bis zu 9 Kohlenstoffatomen, oder ein Benzoylrest ist.

4. Verbindungen nach Anspruch 3, **dadurch gekennzeichnet, daß** R' ein geradkettiger gesättigter Kohlenwasserstoffrest mit 1 bis 9 Kohlenstoffatomen ist.

5. (*E,Z*)-3-Hydroxyimino-6β,7β;15β,16β-dimethylen-17α-pregn-4-en-21,17-carbolacton
(*E*)-3-Hydroxyimino-6β,7β;15β,16β-dimethylen-17α-pregn-4-en-21,17-carbolacton
(*Z*)-3-Hydroxyimino-6β,7β;15β,16β-dimethylen-17α-plegn-4-en-21,17-carbolacton
(*E,Z*)-3-Acetoxyimino-6β,7β;15β,16β-dimethylen-17α-pregn-4-en-21,17-carbolacton
(*E*)-3-Acetoxyimino-6β,7β;15β,16β-dimethylen-17α-pregn-4-en-21,17-carbolacton
(*Z*)-3-Acetoxyimino-6β,7β;15β,16β-dimethylen-17α-pregn-4-en-21,17-carbolacton
(*E,Z*)-3-Propionyloxyimino-6β,7β;15β,16β-dimethylen-17α-pregn-4-en-21,17-carbolacton
*(E)*-3-Propionyloxyimino-6β,7β;15β,16β-dimethylen-17α-pregn-4-en-21,17-carbolacton
(*Z*)-3-Propionyloxyimino-6β,7β;15β,16β-dimethylen-17α-pregn-4-en-21,17-carbolacton
*(E,Z)*-3-Butyryloxyimino-6β,7β;15β,16β-dimethylen-17α-pregn-4-en-21,17-carbolacton
*(E)*-3-Butyryloxyimino-6β,7β;15β,16β-dimethylen-17α-pregn-4-en-21,17-carbolacton
*(Z)*-3-Butyryloxyimino-6β,7β;15β,16β-dimethylen-17α-pregn-4-en-21,17-carbolacton
*(E,Z)*-3-Hexanoyloxyimino-6β,7β;15β,16β-dimethylen-17α-pregn-4-en-21,17-carbolacton
*(E)*-3-Hexanoyloxyimino-6β,7β;15β,16β-dimethylen-17α-pregn-4-en-21,17-carbolacton
*(Z)-*3-Hexanoyloxyimino-6β,7β;15β,16β-dimethylen-17α-pregn-4-en-21,17-carbolacton
*(E,Z)-*3-Nonanoyloxyimino-6β,7β;15β,16β-dimethylen-17α-pregn-4-en-21,17-carbolacton
*(E)*-3-Nonanoyloxyimino-6β,7β;15β,16β-dimethylen-17α-pregn-4-en-21,17-carbolacton
*(,Z)*-3-Nonanoyloxyimino-6β,7β;15β,16β-dimethylen-17α-pregn-4-en-21,17-carbolacton
nach Anspruch 1.

6. Arzneimittel enthaltend eine Verbindung gemäß Anspruch 1 sowie einen pharmakologisch verträglichen Träger.

7. Verwendung der Verbindungen gemäß Anspruch 1, gegebenenfalls in Kombination mit einem oder mehreren Estrogenen, zur Herstellung eines Arzneimittels.

8. Verwendung nach Anspruch 7 zur Herstellung eines Arzneimittels zur Behandlung der Endometriose, zur Behandlung gestagenabhängiger Tumore, zur Behandlung des prämenstruellen Syndroms, zur Behandlung klimakterischer Beschwerden, zur Prävention der Osteoporose, zur Zyklusregulierung und zur Zyklusstabilisierung.

9. Verwendung der Verbindungen gemäß Anspruch 1, gegebenenfalls in Kombination mit einem oder mehreren Estrogenen, zur Herstellung pharmazeutischer Präparate zur Fertilitätskontrolle.

10. Verfahren zur Herstellung der Verbindungen gemäß Anspruch 1 **dadurch gekennzeichnet, daß** man die Verbindung der Formel II 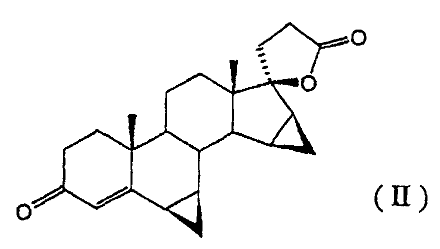 in die 3-Hydroxyiminoverbindungen überführt und gewünschtenfalls diese anschließend durch Veresterung mit einem Carbonsäureanhydrid [(R'C(O)]₂O oder einem Säurehalogenid R'C(O)X (X = Cl, Br; R' hat die in der allgemeinen Formel I angegebene Bedeutung) in Gegenwart einer Base zu den 3-Acyloxyiminoverbin dungen umsetzt.

## Claims

1. An (E,Z) mixture or an isomeric pure (E) and (Z) compound of formula I, 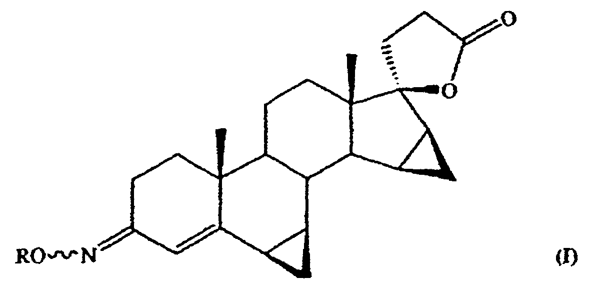 wherein R means a hydrogen atom or an acyl radical with 2 to 10 C atoms.

2. A compound according to claim 1, **characterized in that** R is a hydrogen atom.

3. A compound according to claim 1, **characterized in that** R is C(O)R', wherein R' is a hydrocarbon radical that is straight-chain, branched-chain or cyclic, saturated or unsaturated in up to three places; an alkylcycloalkyl or cycloalkenyl radical, in each case with up to 9 carbon atoms; or a benzoyl radical.

4. A compound according to claim 3, **characterized in that** R' is a straight-chain saturated hydrocarbon radical with 1 to 9 carbon atoms.

5. (E,Z)-3-Hydroxyimino-6β,7β;15β,16β-dimethylene-17α-pregn-4-ene-21,17-carbolactone;
(E)-3-hydroxyimino-6β,7β;15β,16β-dimethylene-17α-pregn-4-ene-21,17-carbolactone;
(Z)-3-hydroxyimino-6β,7β;15β,16β-dimethylene-17α-pregn-4-ene-21,17-carbolactone;
(E,Z)-3-acetoxyimino-6β,7β;15β,16β-dimethylene-17α-pregn-4-ene-21,17-carbolactone;
(E)-3-acetoxyimino-6β,7β;15β,16β-dimethylene-17α-pregn-4-ene-21,17-carbolactone;
(Z)-3-acetoxyimino-6β,7β;15β,16β-dimethylene-17α-pregn-4-ene-21,17-carbolactone;
(E,Z)-3-propionyloxyimino-6β,7β;15β,16β-dimethylene-17α-pregn-4-ene-21,17-carbolactone;
(E)-3-propionyloxyimino-6β,7β;15β,16β-dimethylene-17α-pregn-4-ene-21,17-carbolactone;
(Z)-3-propionyloxyimino-6β,7β;15β,16β-dimethylene-17α-pregn-4-ene-21,17-carbolactone;
(E,Z)-3-butyryloxyimino-6β,7β;15β,16β-dimethylene-17α-pregn-4-ene-21,17-carbolactone;
(E)-3-butyryloxyimino-6β,7β;15β,16β-dimethylene-17α-pregn-4-ene-21,17-carbolactone;
(Z)-3-butyryloxyimino-6β,7β;15β,16β-dimethylene-17α-pregn-4-ene-21,17-carbolactone;
(E,Z)-3-hexanoyloxyimino-6β,7β;15β,16β-dimethylene-17α-pregn-4-ene-21,17-carbolactone;
(E)-3-hexanoyloxyimino-6β,7β;15β,16β-dimethylene-17α-pregn-4-ene-21,17-carbolactone;
(Z)-3-hexanoyloxyimino-6β,7β;15β,16β-dimethylene-17α-pregn-4-ene-21,17-carbolactone;
(E,Z)-3-nonanoyloxyimino-6β,7β;15β,16β-dimethylene-17α-pregn-4-ene-21,17-carbolactone;
(E)-3-nonanoyloxyimino-6β,7β;15β,16β-dimethylene-17α-pregn-4-ene-21,17-carbolactone;
(E,Z)-3-nonanoyloxyimino-6β,7β;15β,16β-dimethylene-17α-pregn-4-ene-21,17-carbolactone, according to claim 1.

6. A pharmaceutical agent comprising a compound according to claim 1 and a pharmacologically compatible vehicle.

7. The use of a compound according to claim 1, optionally in combination with one or more estrogens, for the production of a pharmaceutical agent.

8. The use according to claim 7 for the production of a pharmaceutical agent for the treatment of endometriosis, for the treatment of gestagen-dependent tumors, for the treatment of premenstrual syndrome, for the treatment of menopausal symptoms, for the prevention of osteoporosis, for cycle regulation and for cycle stabilization.

9. The use of the compound according to claim 1, optionally in combination with one or more estrogens, for the production of pharmaceutical preparations for birth control.

10. A process for the production of compounds according to claim 1, **characterized in that** the compound of formula II 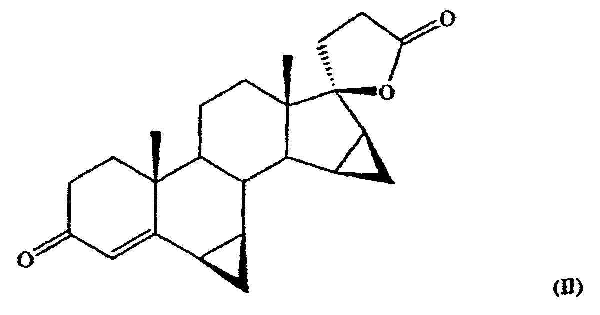 is converted into the 3-hydroxyimino compounds and optionally the latter are then reacted by esterification with a carboxylic anhydride [R'C(O)]₂O or an acid halide R'C(O)X (X = Cl, Br; R' has the meaning indicated in general formula I), in the presence of a base into the 3-acyloxyimino compounds.

## Revendications

1. Mélanges *(E,Z)* et composés *(E)* et *(Z)* isomériquement purs de formule I, 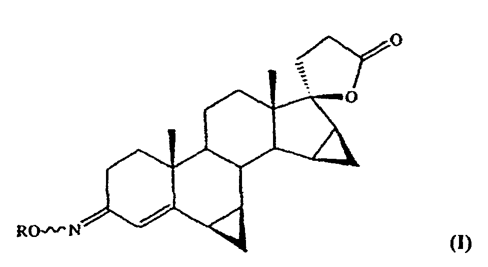 dans laquelle
R représente un atome d'hydrogène ou un radical acyle ayant de 2 à 10 atomes de carbone.

2. Composés selon la revendication 1, **caractérisés en ce que** R représente un atome d'hydrogène.

3. Composés selon la revendication 1, **caractérisés en ce que** R représente un radical C(O)R', dans lequel R' représente un radical hydrocarboné à chaîne linéaire ou ramifiée ou cyclique, saturé ou renfermant jusqu'à trois insaturations, un radical alkylcycloalkyle ou cycloalcényle, ayant dans chaque cas jusqu'à 9 atomes de carbone, ou un radical benzoyle.

4. Composés selon la revendication 3, **caractérisés en ce que** R' représente un radical hydrocarboné à chaîne linéaire, saturé, ayant de 1 à 9 atomes de carbone.

5. (*E,Z*)-3-Hydroxyimino-6β,7β;15β,16β-diméthylène-17α-prégn-4-ène-21,17-carbolactone
*(E)*-3-Hydroxyimino-6β,7β;15β,16β-diméthylène-17α-prégn-4-ène-21,17-carbolactone
*(Z)*-3-Hydroxyimino-6β,7β;15β,16β-diméthylène-17α-prégn-4-ène-21,17-carbolactone
*(E,Z)*-3-Acétoxyimino-6β,7β;15β,16β-diméthylène-17α-prégn-4-ène-21,17-carbolactone
*(E)*-3-Acétoxyimino-6β,7β;15β,16β-diméthylène-17α-prégn-4-ène-21,17-carbolactone
*(Z)*-3-Acétoxyimino-6β,7β;15β,16β-diméthylène-17α-prégn-4-ène-21,17-carbolactone
*(E,Z)*-3-Propionyloxyimino-6β,7β;15β,16β-diméthylène-17α-prégn-4-ène-21,17-carbolactone
*(E)*-3-Propionyloxyimino-6β,7β;15β,16β-diméthylène-17α-prégn-4-ène-21,17-carbolactone
*(Z)*-3-Propionyloxyimino-6β,7β;15β,16β-diméthylène-17α-prégn-4-ène-21,17-carbolactone
*(E,Z)*-3-Butyryloxyimino-6β,7β;15β,16β-diméthylène-17α-prégn-4-ène-21,17-carbolactone
*(E)*-3-Butyryloxyimino-6β,7β;15β,16β-diméthylène-17α-prégn-4-ène-21,17-carbolactone
*(Z)*-3-Butyryloxyimino-6β,7β;15β,16β-diméthylène-17α-prégn-4-ène-21,17-carbolactone
*(E,Z)*-3-Hexanoyloxyimino-6β,7β;15β,16β-diméthylène-17α-prégn-4-ène-21,17-carbolactone
*(E)*-3-Hexanoyloxyimino-6β,7β;15β,16β-diméthylène-17α-prégn-4-ène-21,17-carbolactone
*(Z)*-3-Hexanoyloxyimino-6β,7β;15β,16β-diméthylène-17α-prégn-4-ène-21,17-carbolactone
*(E,Z)*-3-Nonanoyloxyimino-6β,7β;15β,16β-diméthylène-17α-prégn-4-ène-21,17-carbolactone
*(E)*-3-Nonanoyloxyimino-6β,7β;15β,16β-diméthylène-17α-prégn-4-ène-21,17-carbolactone
*(,Z)*-3-Nonanoyloxyimino-6β,7β;15β,16β-diméthylène-17α-prégn-4-ène-21,17-carbolactone
selon la revendication 1.

6. Médicament comprenant un composé selon la revendication 1 ainsi qu'un support pharmacologiquement acceptable.

7. Utilisation des composés selon la revendication 1, éventuellement en combinaison avec un ou plusieurs oestrogènes, pour la fabrication d'un médicament.

8. Utilisation selon la revendication 7 pour la fabrication d'un médicament destiné au traitement de l'endométriose, ou traitement de tumeurs dépendant du progestatif, ou traitement du syndrome prémenstruel, ou traitement des incommodités de la ménopause, à la prévention de l'ostéoporose, à la régulation du cycle menstruel et à la stabilisation du cycle menstruel.

9. Utilisation des composés selon la revendication 1, éventuellement en combinaison avec un ou plusieurs oestrogènes, pour la fabrication de préparations pharmaceutiques destinées au contrôle de la fécondité.

10. Procédé de préparation des composés selon la revendication 1, **caractérisé en ce que** le composé de formule II 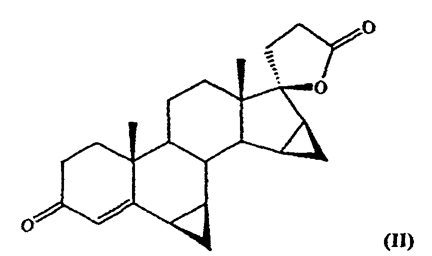 est transformé en composés 3-hydroxyimino et ceux-ci sont ensuite, si on le souhaite, mis à réagir par estérification avec un anhydride carboxylique [(R'C(O)]₂O ou un halogénure d'acide R'C(O)X (X = Cl, Br; R' présente la signification indiquée dans la formule générale I) en présence d'une base, pour donner lieu aux composés 3-acyloxyimino.
